# EUROPÄISCHE PATENTSCHRIFT

(11) **EP 1 076 527 B1**
(45) Veröffentlichungstag und Bekanntmachung des Hinweises auf die Patenterteilung: **17.12.2003**
(21) Anmeldenummer: 98916777.0
(22) Anmeldetag: 07.05.1998
(51) Int. Cl.: A61B 17/64

(54) **BACKE FÜR EINE EXTERNE KNOCHENFIXATIONSVORRICHTUNG**
JAW FOR AN EXTERNAL BONE FIXATION DEVICE
MACHOIRE DESTINEE A UN DISPOSITIF EXTERNE DE FIXATION OSSEUSE

(43) Veröffentlichungstag der Anmeldung: 21.02.2001
(73) Patentinhaber: Synthes AG, Chur, 7002 Chur (CH)
(72) Erfinder: FRIGG, Robert, CH-2544 Bettlach (CH); HEHLI, Markus, CH-7276 Frauenkirch (CH)
(74) Vertreter: Lusuardi, Werther Giovanni, Dr.
(86) Internationale Anmeldenummer: CH9800185
(87) Internationale Veröffentlichungsnummer: WO99058072

(56) Entgegenhaltungen:
- EP-A- 0 593 321
- WO-A-97/41790
- DE-U- 9 320 834

## Beschreibung

Die Erfindung betrifft eine Backe für eine externe Knochenfixationsvorrichtung gemäss dem Oberbegriff des Patentanspruchs 1.

Eine solche Bache ist aus der Druckschrift WO-A-97/41790 bekannt.

Als wichtigste Indikationen für den Einsatz von Fixateurs externes gelten die offenen Frakturen 2. und 3. Grades, infizierte Pseudarthrosen sowie Korrektureingriffe bei Achsenabweichungen und Längendifferenzen.

Aus dem Stand der Technik sind eine Vielzahl von Backen für derartige Fixateurs externes bekannt, welche jedoch alle den Nachteil auswiesen, dass sie äusserst präzise gefertigt werden müssen und dementsprechend teuer sind.

Hier will die Erfindung Abhilfe schaffen. Der Erfindung liegt das Problem zugrunde, eine Backe für eine externe Knochenfixationsvorrichtung zu schaffen, welche dank ihrer einfachen Konstruktion eine anspruchslose Fertigung gestattet, aber trotzdem optimale Resultate garantiert.

Die Erfindung löst die gestellte Aufgabe mit einer Backe, welche die Merkmale des Anspruchs 1 aufweist.

Mit der erfindungsgemässen Backe sind folgende Vorteile erzielbar:
- Die Konstruktion setzt keine engen Fertigungstoleranzen voraus;
- Einsetzbarkeit für sämtliche Fixateur extern Indikationen; dieser versatile Einsatz führt zu einer grösseren Produktionsmenge und vereinfacht vor allem die Logistik, die speziell in Entwicklung- und Schwellenländern problematisch ist;
- Der Backenkörper dient nur zur Aufnahme der zwei Spannelemente (ein Spannelement für den Längsträger und ein Spannelement für die Knochenschraube);
- Beim Fixieren des Längsträgers wird auch die Position des Spannelementes der Knochenschraube fixiert;
- trotz einfacher, fertigungsfreundlicher Konstruktionen besitzt die erfindungsgemässe Backe gleichviel Freiheitsgrade wie konventionelle, teure Backen nach dem Stand der Technik;
- es können Knochenschrauben mit verschiedenem Durchmesser gespannt werden;
- die Konstruktion der Backe ist grössenunabhängig, d.h. das gleiche Backenkonzept kann für Oberschenkel, Unterarmoder Fingerfrakturen angewendet werden; und
- die grosszügigen Toleranzen der verschiedenen Elemente erlauben z.B. auch das Anbiegen des Längsträgers an die Anatomie. Dies ist speziell bei der Versorgung von Beckenfrakturen ein grosser Vorteil. Mit Vorrichtungen nach dem Stand der Technik können Beckenfrakturen nur mit speziell vorgebogenem Längsträger oder mit mehreren Längsträgern, die über Gelenkbacken untereinander verbunden ist, versorgt werden.

Eine bevorzugte Weiterbildung besteht darin, dass die Backe ein Knochenfixationsmittel umfasst, welches vorzugsweise aus einem härteren Material (z.B. Stahl) besteht als der Grundkörper, der z.B. aus Aluminium bestehen kann. Durch die Kombination eines härteren und eines weicheren Materials (z.B. Stahl/Aluminium) ergibt sich eine wesentliche Vereinfachung der Backenkonstruktion. Beim Fixieren der Knochenschraube in der Backe, wird die Knochenschraube in das weiche Aluminium der Backe leicht hineingedrückt. Das gleiche geschieht beim Fixieren des Längsträgers. Damit wird durch die Kompressionsschraube das Spannelement der Knochenschraube, das aus Stahl gefertigt ist, in den weicheren Aluminium-Längsträger eingedrückt. Dank dieser Verformungen wird eine bessere Fixation der Knochenschraube zum Längsträger erreicht, als wenn die Fixation nur durch Reibung erzielt würde. Bei Vorrichtungen nach dem Stand der Technik wird eine formschlüssige Verbindung durch Verzahnung der verschiedenen Spannelemente erreicht, was stark erhöhte Produktionskosten zur Folge hat. Die Kombination von Stahl mit Aluminium hat den zusätzlichen Vorteil, dass die Backen mehrmals verwendet werden können. Da die Gewindeteile aus Stahl gefertigt sind, ist die Gefahr einer Überbeanspruchung oder eines Festfressens der Gewinde vernachlässigbar.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine perspektivische Darstellung der Backe;
Fig. 2 eine Vorderansicht des Grundkörpers der Backe nach Fig. 1;
Fig. 3 einen parallel zur Vorderfläche des Grundkörpers verlaufenden Querschnitt;
Fig. 4 eine Seitenansicht des Grundkörpers der Backe nach Fig. 1;
Fig. 5 einen parallel zur Seitenfläche des Grundkörpers verlaufenden Querschnitt; und
Fig. 6 einen Längsschnitt durch die Zugschraube der Backe nach Fig. 1.

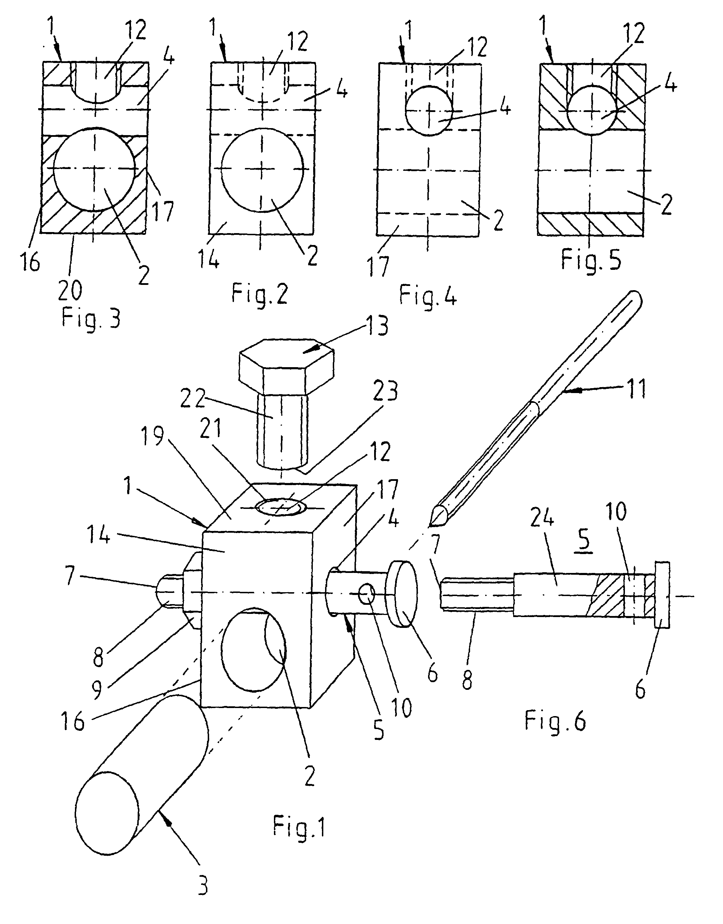

Die in den Fig. 1 - 5 dargestellte Backe für eine externe Knochenfixationsvorrichtung umfasst im wesentlichen einen quaderförmigen, vorzugsweise aus Aluminium gefertigten Grundkörper 1 mit einer Vorderfläche 14, einer Hinterfläche 15, zwei Seitenflächen 16 und 17, einer Hinterfläche 18 einer Deckfläche 19 und einer Basisfläche 20.

Der Grundkörper 1 weist eine erste - von der Vorderfläche 14 zur Hinterfläche 15 verlaufende - durchgehenden Bohrung 2 zur Aufnahme eines Längsträgers 3 auf. Der Längsträger 3 kann voll oder hohl ausgebildet sein und seine Oberfläche kann glatt oder aufgerauht sein. Vorzugsweise ist der Längsträger 3 aus Aluminium gefertigt.

Der Grundkörper 1 weist eine zweite - von der Seitenfläche 16 zur Seitenfläche 17 verlaufende - durchgehende Bohrung 4 auf, welche senkrecht zur ersten Bohrung 2 verläuft und diese, wie in den Fig. 2 - 5 ersichtlich, partiell durchdringt.

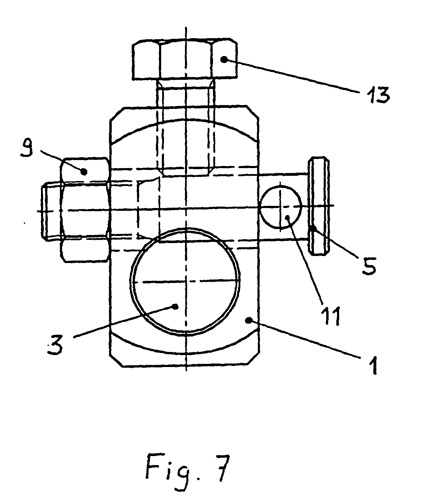

In der zweiten Bohrung 4 ist, wie in Fig. 1 und 7 dargestellt, eine, einen Kopf 6 aufweisende Zugschraube 5 (Fig. 6) mit ihrem freien, ein Gewinde 8 aufweisendes Ende 7 eingeführt. Die Zugschraube 5 wird einerseits durch ihren, einen grösseren Durchmesser als die zweite Bohrung 4 aufweisenden Kopf 6 gesichert, der einen Anschlag auf der Seitenfläche 17 des Grundkörper 1 bildet; anderseits wird die Zugschraube 5 durch die auf ihr freies Ende 7 aufgeschraubte Mutter 9 gesichert, welche einen Anschlag an der gegenüberliegenden Seitenfläche 16 des Grundkörper 1 bildet. Die Zugschraube 5 ist vorzugsweise aus Stahl gefertigt.

Die Zugschraube 5 ist in ihrem kreiszylindrischen Schaftteil 24 - unterhalb des Kopfes 6 - mit einer Querbohrung 10 versehen, welche ein Knochenfixationsmittel 11, z.B. eine Schanzsche Schraube aufnehmen kann.
Nach Einführung des Knochenfixationsmittels 11 in die Querbohrung 10 kann die Zugschraube 5 mittels der Mutter 9 nach links verschoben werden, bis die Querbohrung 10 in den Bereich der zweiten Bohrung 4 gelangt und dadurch das Knochenfixationsmittels 11 an der Seitenfläche 17 des Grundkörpers 1 zur Anlage kommt, wie dies in Fig. 7 dargestellt ist.

Vorteilhafterweise wählt man ein Knochenfixationsmittel 11, welches aus einem härteren Material (z.B. Stahl) besteht als der Grundkörper 1, damit eine verbesserte Klemmwirkung erzielt werden kann.

Der Grundkörper 1 weist im weiteren eine Gewindebohrung 12 mit Gewinde 21 auf, welche sowohl zur ersten, als auch zur zweiten durchgehenden Bohrung 2 und 4 senkrecht verläuft und von der Deckfläche 19 zur zweiten durchgehende Bohrung 4 verläuft und in letztere mündet. Sie dient zur Aufnahme einer Fixationsschraube 13 mit Gewinde 22, welche mit ihrem freien Ende 23 an der in die zweite Bohrung 4 eingeführten Zugschraube 5 zur Anlage gebracht werden kann. Da der Durchmesser des Schaftteils 24 der Zugschraube 5 kleiner als der Durchmesser der zweiten Bohrung 4 ist lässt sich die Zugschraube 5 mittels der Fixationsschraube 13 in den Bereich der ersten Bohrung 2 drücken, wo sie mit dem darin eingeführten Längsträger 3 zur tangentialen Anlage kommt, so dass der Längsträger 3 gegenüber dem Grundkörper 1 axial und rotativ fixiert wird.

## Patentansprüche

1. Backe für eine externe Knochenfixationsvorrichtung, wobei
A) die Backe einen quaderförmigen Grundkörper (1) und eine Zugschraube (5) mit einem Kopf (6) und einer Mutter (9) aufweist; **dadurch gekennzeichnet, dass**
B) der Grundkörper (1) eine erste durchgehenden Bohrung (2) zur Aufnahme eines Längsträgers (3) aufweist;
C) der Grundkörper (1) eine zweite, durchgehende Bohrung (4) aufweist, welche senkrecht zur ersten Bohrung (2) verläuft und diese partiell durchdringt;
D) in die zweite Bohrung (4) die, den Kopf (6) aufweisende Zugschraube (5) mit ihrem freien Ende (7) einführbar ist, wobei der Kopf (6) einen Anschlag am Grundkörper (1) bildet;
E) die Zugschraube (5) an ihrem freien Ende (7) eine Gewinde (8) zur Aufnahme der Mutter (9) aufweist, welche einen Anschlag am Grundkörper (1) bildet;
F) die Zugschraube (5) unterhalb ihres Kopfes (6) eine Querbohrung (10) zur Aufnahme eines Knochenfixationsmittels (11) aufweist;
G) die Querbohrung (10) mittels der Mutter (9) in den Bereich der zweiten Bohrung (4) bringbar ist, wobei ein in die Querbohrung (1) eingeführtes Knochenfixationsmittel (11) am Grundkörper (1) zur Anlage bringbar ist;
H) der Grundkörper (1) eine Gewindebohrung (12) aufweist, welche sowohl zur ersten, als auch zur zweiten durchgehenden Bohrung (2,4) senkrecht verläuft und in die zweite durchgehende Bohrung (4) mündet, zur Aufnahme einer Fixationsschraube (13);
I) der Durchmesser der Zugschraube (5) kleiner als Durchmesser der zweiten Bohrung (4) ist;
K) die Zugschraube (5) mittels der Fixationsschraube (13) in den Bereich der zweiten Bohrung (2) mit dem Längsträger (3) zur tangentialen Anlage bringbar ist.

2. Backe nach Anspruch 1, **dadurch gekennzeichnet, dass** sie eine Fixationsschraube (13) umfasst.

3. Backe nach einem der Ansprüche 1 bis 2, **dadurch gekennzeichnet, dass** sie ein Knochenfixationsmittels (11) umfasst, welches vorzugsweise aus einem härteren Material besteht als der Grundkörper (1).

4. Backe nach einem der Ansprüche 1 bis 3, **dadurch gekennzeichnet, dass** der Grundkörper (1) aus Aluminium besteht.

5. Backe nach einem der Ansprüche 2 bis 4, **dadurch gekennzeichnet, dass** die Zugschraube (5) aus einem härteren Material als der Grundkörper (1) besteht und vorzugsweise aus Stahl gefertigt ist.

6. Backe nach einem der Ansprüche 2 bis 5, **dadurch gekennzeichnet, dass** die Zugschraube (5) einen zwischen dem Gewinde (8) und dem Kopf (6) liegenden, gewindefreien Schaftteil (24) aufweist.

7. Backe nach einem der Ansprüche 2 bis 6, **dadurch gekennzeichnet, dass** sie einen Längsträger (3) umfasst, der vorzugsweise aus einem weicheren Material gefertigt ist als die Zugschraube (5).

## Claims

1. A clamp for an external bone fixation device, whereby
A) The clamp comprises a parallelepipedic basic body (1) and a tension bolt (5) with a head (6) and a nut (9),
**characterised in that**
B) The basic body (1) is fitted with a first and continuous borehole (2) to receive a longitudinal support (3);
C) The basic body (1) is fitted with a second and continuous borehole (4) running perpendicularly to the first borehole (2) and partly crossing it;
D) The tension bolt (5) comprises the head (6) and can be inserted by its free end (7) into the second borehole (4), the head (6) forming a stop against the basic body (1 );
E) The tension bolt (5) is fitted at its free end (7) with a thread (8) to engage the nut (9) forming a stop against the basic body (1 );
F) The tension bolt (5) is fitted with a transverse borehole (10) underneath its head (6) to receive a bone fixation device (11 );
G) The transverse borehole (10) can be moved by the nut (9) into the zone of the second borehole (4), a bone fixation device (11 ) being displaceable in this process to come to rest against the basic body (1);
H) The basic body (1) is fitted with a threaded borehole (12) running perpendicularly to both first and second continuous bore holes (2, 4) and terminating in the second borehole (4) and receiving a fixation screw (13);
I) The diameter of the tension bolt (5) is less than the diameter of the second borehole (4);
K) The tension bolt (5) can be moved by the fixation screw (13) into the zone of the second borehole (2) to be tangentially resting against the longitudinal support (3).

2. Clamp as defined in claim 1 **characterized in that** it comprises a fixation screw (13).

3. Clamp as claimed in claim 1 or 2, **characterized in that** it comprises a bone fixation device (11) preferably made of a material harder than the basic body (1).

4. Clamp as claimed in one of claims 1 through 3, **characterized in that** the basic body (1) is made of aluminum.

5. Clamp as claimed in one of claims 1 through 4, **characterized in that** the tension bolt (5) is made of a material harder than that of the basic body (1) and preferably of steel.

6. Clamp as claimed in one of claims 1 through 5, **characterized in that** the tension bolt (5) is fitted with an unthreaded shank part (24) located between the thread (8) and the head (6).

7. Clamp as claimed in one of claims 1 through 6, **characterized in that** it comprises a longitudinal support (3) made preferably of a softer material than the tension bolt (5).

## Revendications

1. Mâchoire pour un dispositif externe de fixation osseuse dans laquelle
a) la mâchoire présente un corps de base (1) carré et une vis de tension (5) avec une tête (6) et un écrou (9), **caractérisée en ce que**
b) le corps de base (1) présente un premier orifice (2) de passage pour accueillir un support longitudinal (3),
b) le corps de base (1) présente un deuxième orifice (4) de passage qui s'étend perpendiculairement au premier orifice (2) et traverse partiellement celui-ci,
d) la vis de tension (5) qui présente la tête (6) peut être introduite avec son extrémité libre (7) dans le deuxième orifice (4), la tête (6) formant une butée sur le corps de base (1),
e) la vis de tension (5) présente à son extrémité libre (7) un filetage (8) pour accueillir l'écrou (9), qui forme une butée sur le corps de base,
f) la vis de tension (5) présente en dessous de sa tête (6) un orifice transversal (10) pour accueillir un moyen de fixation osseuse (11 ),
g) l'orifice transversal (10) peut être amené au moyen de l'écrou (9) dans la région du deuxième orifice (4), un moyen de fixation osseuse (11) introduit dans l'orifice transversal (10) pouvant être mis en contact avec le corps de base (1),
h) le corps de base (1) présente un orifice fileté (12) qui s'étend perpendiculairement aussi bien au premier qu'au deuxième orifice de passage (2, 4) et débouche dans le deuxième orifice (4) de passage pour accueillir une vis de fixation (13),
i) le diamètre de la vis de tension (5) est plus petit que le diamètre du deuxième orifice (4),
k) la vis de tension (5) peut être mise en contact tangentiel avec le support longitudinal (3) dans la région du deuxième orifice (2) au moyen de la vis de fixation (13).

2. Mâchoire selon la revendication 1, **caractérisée en ce qu'**elle comprend une vis de fixation (13).

3. Mâchoire selon une des revendications 1 à 2, **caractérisée en ce qu'**elle comprend un moyen de fixation osseuse (11) qui est constitué, de préférence, d'un matériau plus dur que le corps de base (1).

4. Mâchoire selon une des revendications 1 à 3, **caractérisée en ce que** le corps de base (1) est constitué d'aluminium.

5. Mâchoire selon une des revendications 2 à 4, **caractérisée en ce que** la vis de tension (5) est constituée d'un matériau plus dur que le corps de base et est fabriquée, de préférence, en acier.

6. Mâchoire selon une des revendications 2 à 5, **caractérisée en ce que** la vis de tension (5) présente une partie tige (24) sans filetage se trouvant entre le filetage (8) et la tête (6).

7. Mâchoire selon une des revendications 2 à 6, **caractérisée en ce qu'**elle comprend un support longitudinal (3) qui est fabriqué, de préférence, dans un matériau plus mou que la vis de tension (5).
